# EUROPEAN PATENT APPLICATION

(11) **EP 1 048 296 A1**
(43) Date of publication of application: **02.11.2000**
(21) Application number: 98959167.2
(22) Date of filing: 11.12.1998
(51) Int. Cl.: A61K 31/47, C07D 215/56, C07C 231/02, C07C 233/54, C07C 327/42

(54) **DRUGS AUGMENTING NKT CELLS**

(30) Priority: 15.12.1997 JP 34540997
(71) Applicant: FUJISAWA PHARMACEUTICAL CO., LTD., Osaka-shi Osaka 541-8514 (JP)
(72) Inventor: MASUNAGA, Taro, Kobe-shi, Hyogo 655-0049 (JP); SASAGAWA, Yuka, Suita-shi, Osaka 565-0875 (JP); SEKI, Nobuo, Takarazuka-shi, Hyogo 665-0877 (JP); TSUJI, Kiyoshi, Kishiwada-shi, Osaka 596-0831 (JP); SPEARS, Glen, W., Ikeda-shi, Osaka 563-0026 (JP); TOJO, Takashi, Osaka-shi, Osaka 537-0011 (JP)
(74) Representative: Gille Hrabal Struck Neidlein Prop Roos
(86) International application number: JP9805604
(87) International publication number: WO9930711

(57) **Abstract**

There is provided a NKT cell augmentation-mediated autoimmunosuppressant and/or potentiator on normal immune responses characterized by its comprising a compound of the following general formula or a pharmaceutically acceptable salt thereof as an active ingredient. 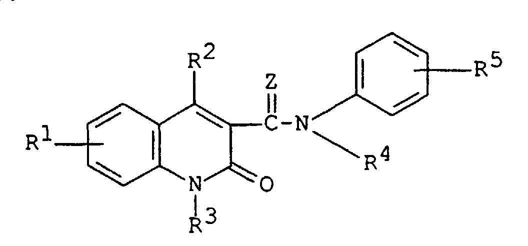
[wherein R¹ represents hydrogen or halogen,
R² represents hydroxy,
R³ represents lower alkyl,
R⁴ represents lower alkyl,
R⁵ represents hydrogen or lower alkoxy, and
Z represents O or S]

Also provided is a novel process for synthesizing the above compound.

## Description

### TECHNICAL FIELD

This invention relates to a NKT cell augmentation-mediated autoimmunosuppressant and/or potentiator on normal immune responses comprising a compound of the general formula (I) shown hereinafter or a pharmaceutically acceptable salt thereof as an active ingredient.

Furthermore, this invention relates to a new process for synthesizing a compound of the general formula (I) or a pharmaceutically acceptable salt thereof.

### BACKGROUND ART

Of the 1,2-dihydroquinoline compound of this invention, which may be represented by the general formula (I) presented below, some species are known compounds as described in WO95/24395 and EP 0059698 and have been shown to have inhibitory activity on the leakage of a proteinuria, inhibitory activity on anti-TNP IgM production, effect on carrageenan edema in rats, or effect on adjuvant arthritis in rats, among other activities. However, it has not been known that those compounds ever have the property to augment NKT cells.

WO95/24395 further discloses the following process for synthesizing the 1,2-dihydroquinoline compound (I), taking 6-chloro-1,2-dihydro-4-hydroxy-1-methyl-2-oxo-3-[N-phenyl-N-methyl(thiocarbamoyl)]quinoline (Ia) as an example.

### (Conventional process)

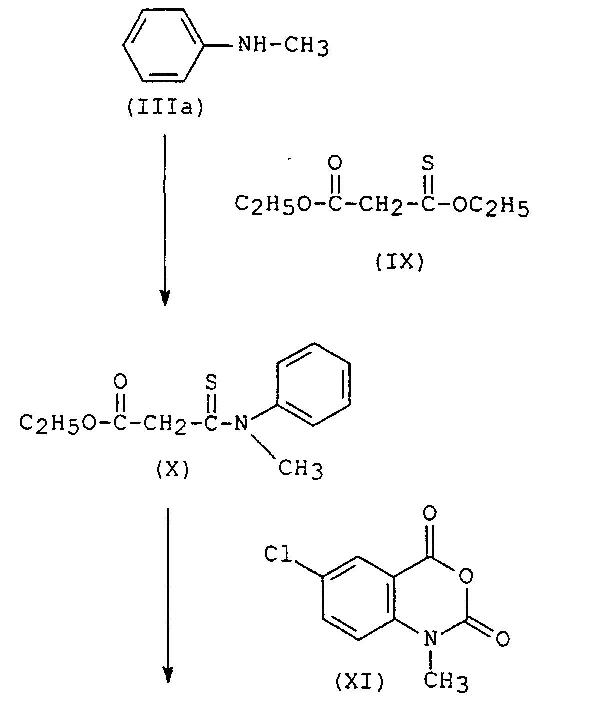

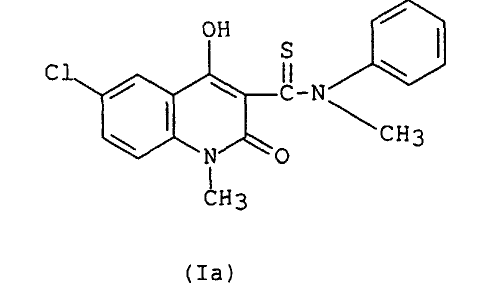

In the conventional process, compound (Ia) is produced from compound (IIIa) by reacting compound (IIIa) with compound (IX) under reflux conditions to give the compound (X) in a yield of 40.8% in the first place and then reacting this compound (X) with compound (XI) in the presence of a strong base such as sodium hydride to give the objective compound (Ia) in a yield of 17.5%. According to this technology, the total yield of the objective compound (Ia) based on compound (IIIa) is 7.1%. The production technology proposed by the above Patent laid-open for the 1,2-dihydroquinoline compound (Ia), which is subsumed in the compound of general formula (I), has shortcomings such as low yields in the respective steps.

### DISCLOSURE OF INVENTION

This invention relates to an NKT cell augmentation-mediated autoimmunosuppressant and/or potentiator on normal immune responses characterized by comprising a compound of the following general formula (I) or a pharmaceutically acceptable salt thereof as an active ingredient. 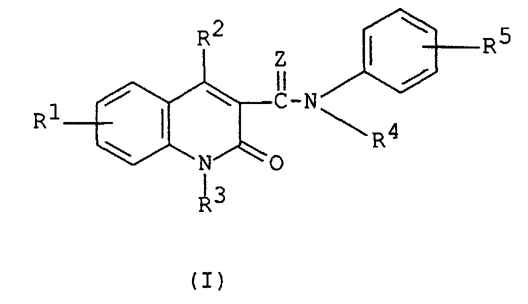
[wherein R¹ represents hydrogen or halogen,
R² represents hydroxy,
R³ represents lower alkyl,
R⁴ represents lower alkyl,
R⁵ represents hydrogen or lower alkoxy, and
Z represents O or S]

This invention further provides the following process for producing said 1,2-dihydroquinoline compound (I) or a salt thereof, which is superior to the known process in yield, etc.

The process for producing the said 1,2-dihydroquinoline compound (I) according to this invention is as follows.

### Process (1)

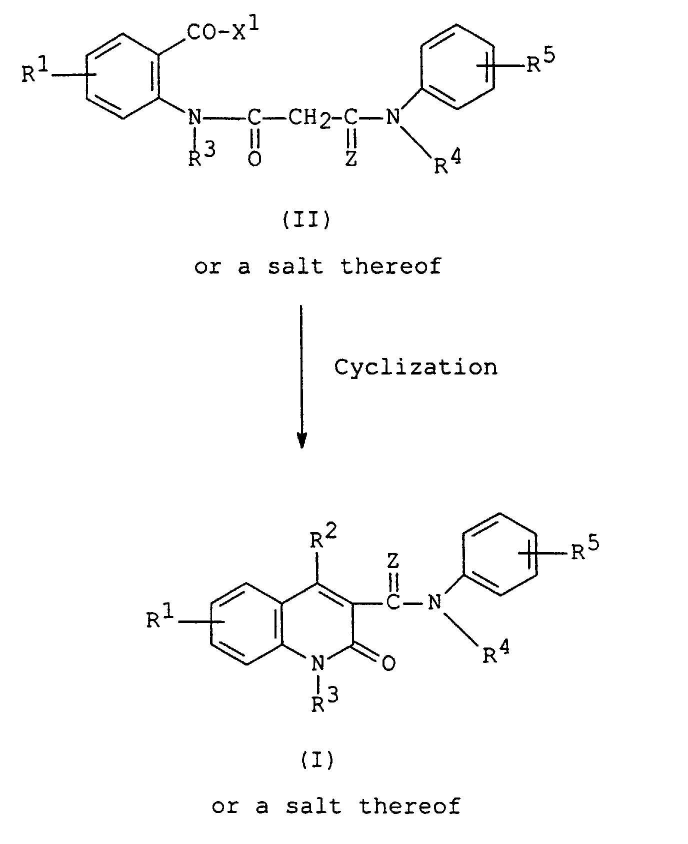

[wherein, R¹, R², R³, R⁴, R⁵ and Z are each as defined above; X¹ represents a leaving group]

The starting compound (II) can be produced by the following processes.

### Process (A)

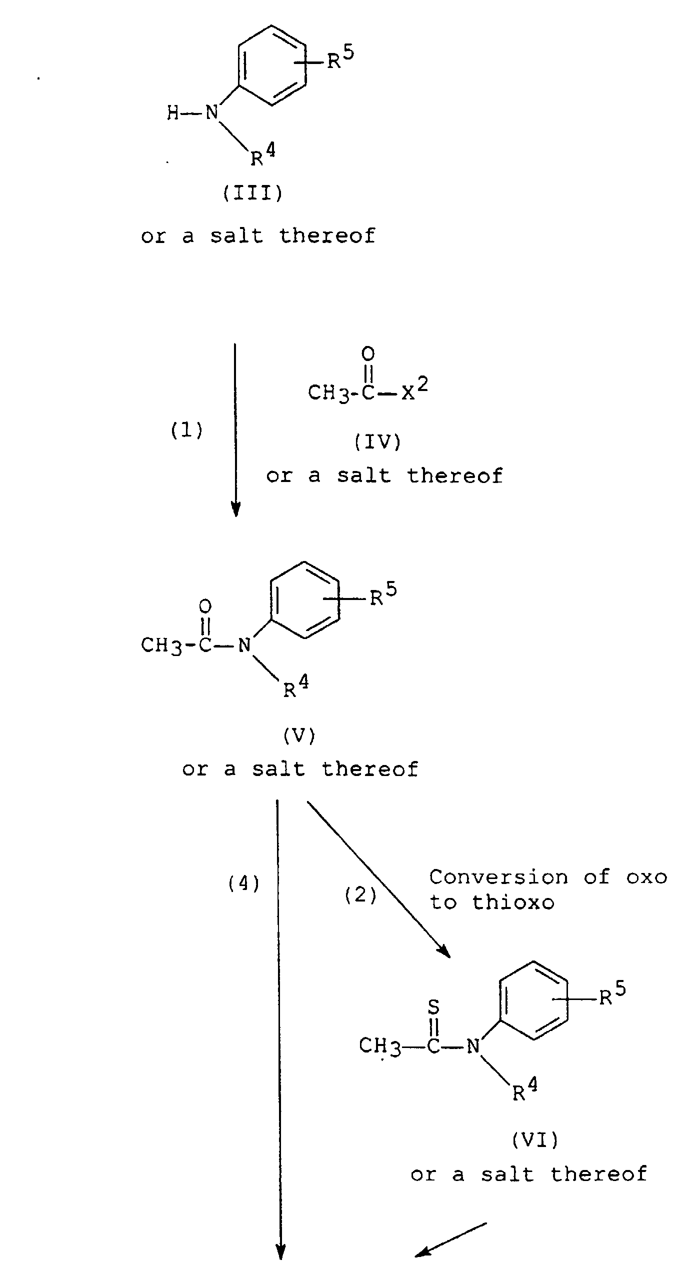

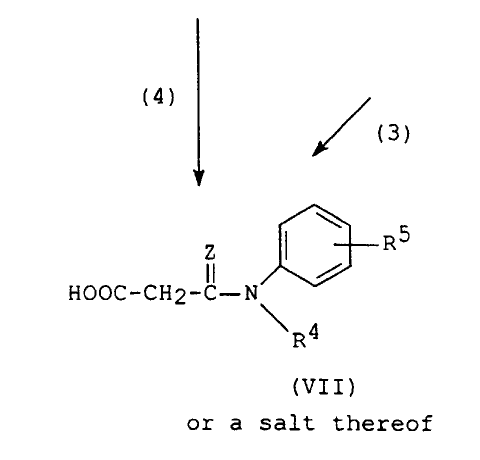

### Process (B)

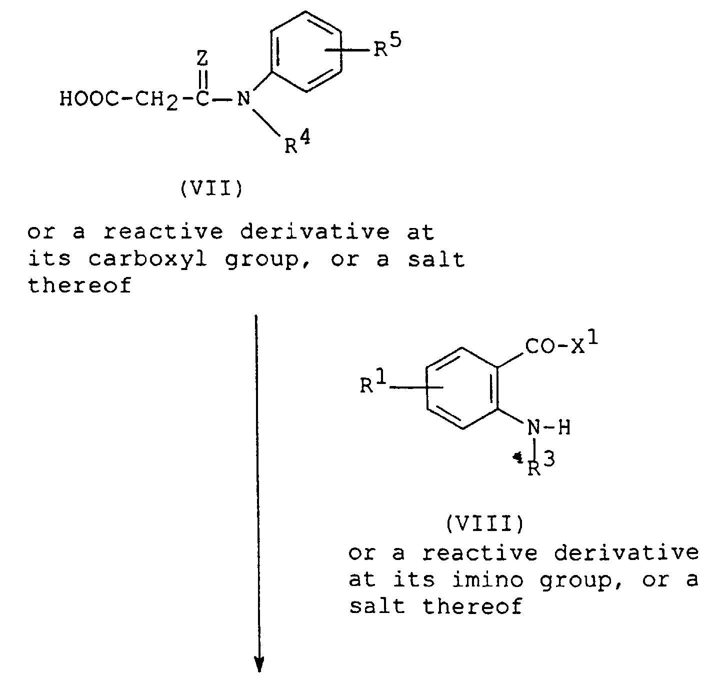

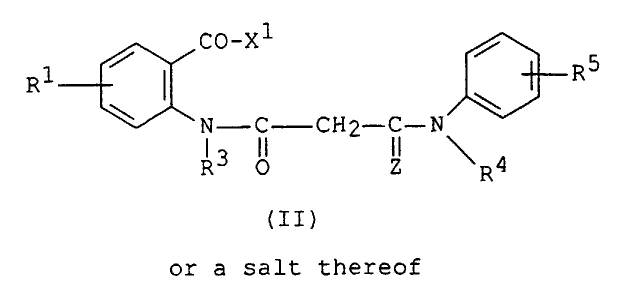

[wherein, R¹, R³, R⁴, R⁵, X¹ and Z are each as defined above; X² represents a leaving group]

In the above and subsequent descriptions of the present specification, suitable example and illustration of the various definitions, which the present invention intends to include within the scope thereof, are explained in detail as follows.

Suitable pharmaceutically acceptable salts of the object compound (I) are conventional non-toxic salts and may include e.g. a salt with a base or an acid addition salt such as a salt with an inorganic base, for example, an alkali metal salt (e.g. sodium salt, potassium salt, etc.), an alkaline earth metal salt (e.g. calcium salt, magnesium salt, etc.), an ammonium salt; a salt with an organic base, for example, an organic amine salt (e.g. triethylamine salt, pyridine salt, picoline salt, ethanolamine salt, triethanolamine salt, dicyclohexylamine salt, N,N'-dibenzylethylenediamine salt, etc.); an inorganic acid addition salt (e.g. hydrochloride, hydrobromide, sulfate, phosphate, etc.); an organic carboxylic or sulfonic acid addition salt (e.g. formate, acetate, trifluoroacetate, maleate, tartrate, methanesulfonate, benzenesulfonate, toluenesulfonate, etc.); a salt with a basic or acidic amino acid (e.g. arginine, aspartic acid, glutamic acid, etc.).

The term "lower" is used to intend a group having 1 to 6, preferably 1 to 4, carbon atoms, unless otherwise provided.

Suitable "lower alkyl" may include straight or branched one such as methyl, ethyl, propyl, isopropyl, butyl, t-butyl, pentyl, hexyl, etc., in which more preferred example may be C₁∼C₄ alkyl groups.

Suitable "lower alkoxy" may include methoxy, ethoxy, propoxy, isopropoxy, butoxy, isobutoxy, t-butoxy, pentyloxy, t-pentyloxy, hexyloxy, etc., in which more preferred example may be C₁∼C₄ alkoxy.

Suitable "halogen" may include fluoro, chloro, bromo and iodo.

Suitable "leaving group" may include halogen (e.g. fluoro, chloro, bromo and iodo); lower alkoxy (e.g. methoxy, ethoxy, propoxy, etc.); and sulfonyloxy ( e.g. methylsulfonyloxy, phenylsulfonyloxy, etc.) and the like.

The preferred embodiments of the compound (I) according to this invention are as follows.
6-Chloro-1,2-dihydro-4-hydroxy-1-methyl-2-oxo-3-[N-phenyl-N-methyl(thiocarbamoyl)]quinoline
6-Chloro-1,2-dihydro-4-hydroxy-1-methyl-2-oxo-3-[N-methyl-N-(4-methoxyphenyl)(thiocarbamoyl)]-quinoline
1,2-Dihydro-4-hydroxy-1-methyl-2-oxo-3-[N-phenyl-N-methylcarbamoyl]quinoline

The process for producing the compound (I) of this invention and that for producing the compound (II), which is of use as an intermediate, are now explained in detail in the following.

### Process (1)

The compound (I) or a salt thereof can be produced by subjecting the compound (II) or a salt thereof to cyclization reaction.

This reaction is usually conducted in the common solvent, such as water, alcohol (e.g. methanol, ethanol, etc.), benzene, N,N-dimethylformamide, tetrahydrofuran, toluene, methylene chloride, ethylene dichloride, chloroform, dioxane, or diethyl ether or an arbitrary other solvent that dose not adversely affect the reaction or in a mixture of such solvents.

The reaction temperature is not critical and the reaction is usually carried out under cooling to heating.

This reaction is usually carried out by the procedure using an inorganic or organic base, such as alkali metals (e.g. sodium, potassium, etc.), alkali metal hydroxides (e.g. sodium hydroxide, potassium hydroxide, etc.), alkali metal hydrogencarbonates (e.g. sodium hydrogencarbonate, potassium hydrogencarbonate, etc.), alkali metal carbonates (e.g. sodium carbonate, potassium carbonate, etc.), tri(lower)alkylamines (e.g. trimethylamine, triethylamine, diisopropylethylamine, etc.), alkali metal hydrides (e.g. sodium hydride, etc.), alkali metal (lower)alkoxides (e.g. sodium methoxide, sodium ethoxide, etc.), pyridine, lutidine, picoline, dimethylaminopyridine, N-(lower) alkylmorpholines, N,N-di(lower)alkylbenzylamines, N,N-di(lower)-alkylanilines, and so on.

When the base and/or the starting compound is a liquid, it can be used as the solvent as well.

### Process (A)-(1)

The compound (V) or a salt thereof can be produced by reacting the compound (III) or a salt thereof with the compound (IV) or a salt thereof.

This reaction can be carried out in the manner disclosed in Preparation 1-(1) to be described below or similar manners thereto.

### Process (A)-(2)

The compound (VI) or a salt thereof can be produced by subjecting compound (V) or a salt thereof to a reaction for conversion of oxo to thioxo.

This reaction can be carried out in the manner disclosed in Preparation 1-(2) or 2 to be described below or similar manners thereto.

### Process (A)-(3)

The compound (VII) or a salt thereof can be produced by introducing a carboxy group into the compound (VI) or a salt thereof.

This reaction can be carried out in the manner disclosed in Preparation 3 or 4 to be described below or similar manners thereto.

### Process (A)-(4)

The compound (VII) or a salt thereof can be produced by introducing a carboxy group into the compound (V) or a salt thereof.

This reaction can be carried out in the manner disclosed in Preparation 3 or 4 to be described below or similar manners thereto.

### Process (B)

The compound (II) or a salt thereof can be produced by reacting the compound (VII) or a reactive derivative at its carboxyl group, or a salt thereof, with compound (VIII) or a reactive derivative at its imino group, or a salt thereof.

Suitable reactive derivative at the imino group of the compound (VIII) includes silyl derivatives obtainable by reacting compound (VIII) with a silyl compound such as N,O-bis(trimethylsilyl)acetamide, N-trimethylsilylacetamide or the like; and derivatives obtainable by reacting compound (VIII) with phosphorus trichloride or phosgene.

Suitable reactive derivative at the carboxyl group of the compound (VII) includes acid halides, acid anhydrides and active esters. Suitable example may be the acid chloride; acid azide; mixed acid anhydrides with various acids such as substituted phosphoric acid (e.g. dialkylphosphoric acids, phenylphosphoric acid, diphenylphosphoric acid, dibenzylphosphoric acid, halogenated phosphoric acids, etc.), dialkylphosphorous acids, sulfurous acid, thiosulfuric acid, alkanesulfonic acids (e.g. methanesulfonic acid, ethanesulfonic acid, etc.), sulfuric acid, alkylcarbonic acids, aliphatic carboxylic acids (e.g. pivalic acid, pentanoic acid, isopentanoic acid, 2-ethylbutyric acid, trichloroacetic acid, etc.), aromatic carboxylic acids (e.g. benzoic acid etc.), etc.; symmetric acid anhydride; active amides with imidazole, 4-substituted imidazole, dimethylpyrazole, triazol, tetrazole, etc.; active esters (e.g. cyanomethyl ester, methoxymethyl ester, dimethyliminomethyl [(CH₃)₂N⁺=CH-] ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenylthio ester, p-nitrophenylthio ester, p-cresylthio ester, carboxymethylthio ester, pyranyl ester, pyridyl ester, piperidyl ester, 8-quinolylthio ester, etc.); and esters with N-hydroxy compounds (e.g. N,N-dimethylhydroxylamine, 1-hydroxy-2(1H)-pyridone, N-hydroxysuccinimide, N-hydroxybenzotriazole, N-hydroxyphthalimide, 1-hydroxy-6-chloro-1H-benzotriazole and so on.

These reactive derivatives can be optionally selected from them according to the kind of the compound (VII) to be used.

This reaction is usually carried out in the common solvent, such as acetone, dioxane, acetonitrile, chloroform, methylene chloride, ethylene chloride, tetrahydrofuran, ethyl acetate, N,N-dimethylformamide, pyridine, or the like, any other organic solvent that does not interfere with the reaction, or a mixture of such solvents.

The reaction is preferably conducted in the presence of a dehydrating agent such as molecular sieves 3Å or 4Å.

When compound (VII) is reacted in the free acid form or in the form of a salt, the reaction is preferably conducted in the presence of the conventional condensing agent, such as N,N'-dicyclohexyl-carbodiimide; N-cyclohexyl-N'-morpholinoethyl-carbodiimide; N-cyclohexyl-N'-(4-diethylamino-cyclohexyl)carbodiimide, N,N'-diisopropylcarbodiimide; N-ethyl-N'-(3-dimethylaminopropyl)carbodiimide; N,N'-carbonylbis(2-methylimidazole); pentamethyleneketene-N-cyclohexylimine; diphenylketene-N-cyclohexylimine; ethoxyacetylene; 1-alkoxy-1-chloroethylenes; trialkyl phosphites, isopropyl polyphosphate; phosphorus oxychloride (phosphoryl chloride); phosphorus trichloride; thionyl chloride; oxalyl chloride; triphenylphosphine; 2-ethyl-7-hydroxybenzisooxazolium salt; 2-ethyl-5-(m-sulfophenyl)isoxazolium hydroxide intra-molecular; 1-(p-chlorobenzenesulfonyloxy)-6-chloro-1H-benzotriazole; and the so-called Vilsmeier reagents prepared by reacting N,N-dimethylformamide with thionyl chloride, phosgene, phosphorus oxychloride and so on.

This reaction may be carried out in the presence of an organic or inorganic base such as alkali metal hydrogencarbonates, tri(lower)alkylamines, pyridine, N-lower alkylmorpholines, N,N-di(lower)alkylbenzylamines and so on.

The reaction temperature is not critical, and the reaction is usually carried out under cooling to heating.

The novel method of synthesis according to this invention is now described in detail, taking the synthesis of 6-chloro-1,2-dihydro-4-hydroxy-1-methyl-2-oxo-3-[N-phenyl-N-methyl(thiocarbamoyl)]quinoline (Ia) as an example.

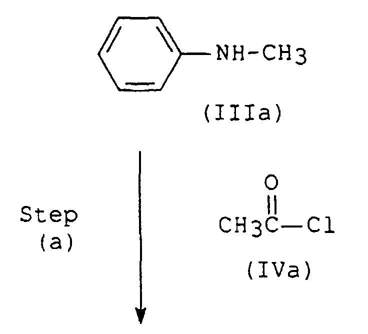

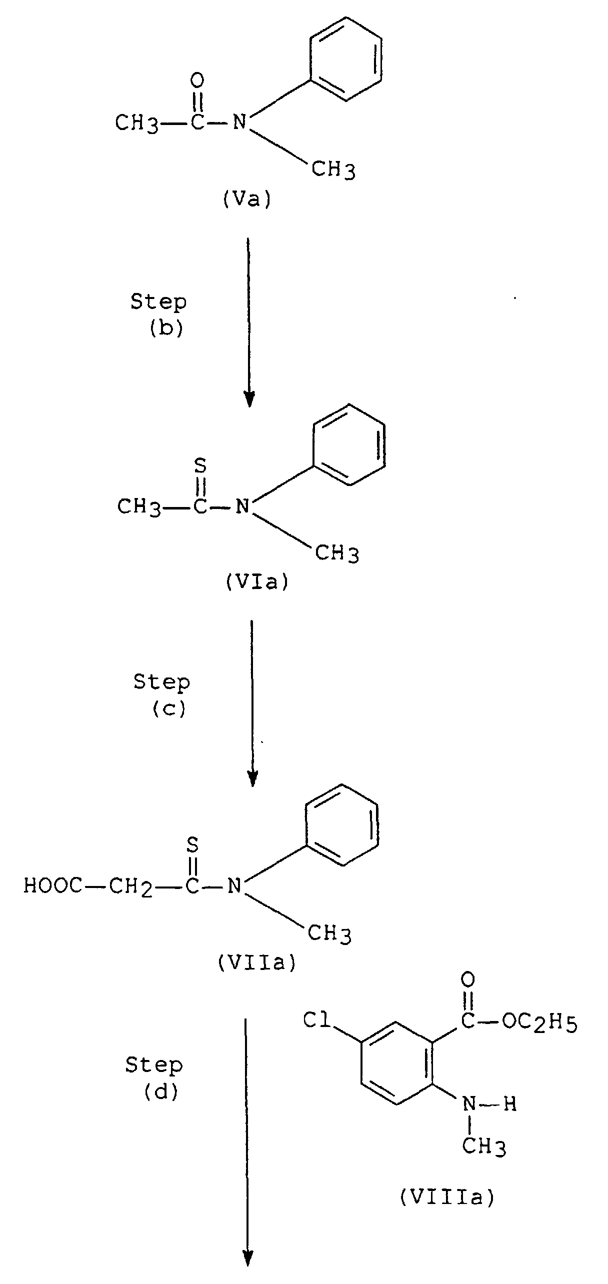

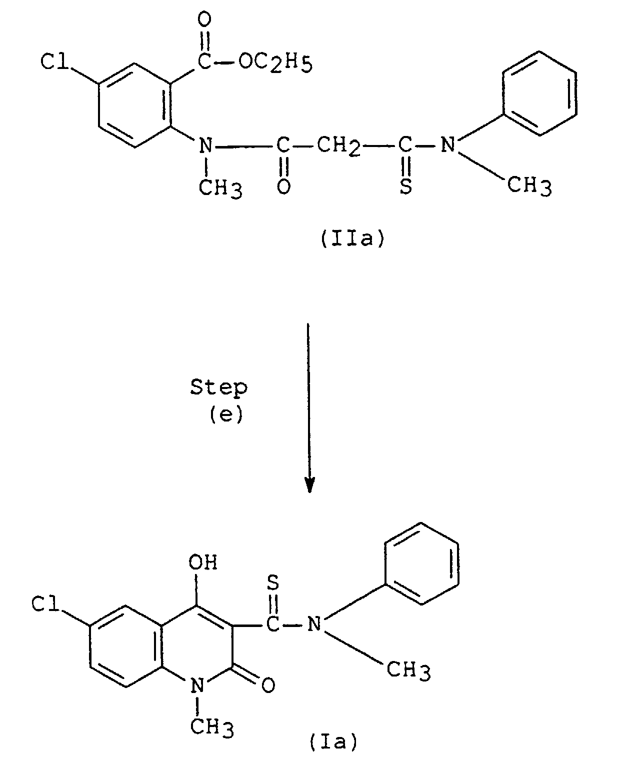

According to the novel method of synthesis according to this invention, the objective compound could be obtained in high stepwise yields, namely 90.2% in Step (a), 73.2% in Step (b), 66.4% in Step (c), 86.0% in Step (d) and 96.9% in Step (e). The total yield from the compound (IIIa) to the objective compound (Ia) was 36.5%, which represents a marked improvement over the total yield [(IIIa)∼(Ia)] of 7.1% mentioned in the above Patent laid-open (WO95/24395). The respective steps of the method are described in detail in Preparations and Examples presented hereinafter [Step (a) in Preparation 1-(1), Step (b) in Preparation 1-(2), Step (c) in Preparation 3, Step (d) in Preparation 5, and step (e) in Example 1].

NKT cells are unique cells having not only T-cell receptors but also NK cell receptors and, as such, have the characteristics of the two distinct kinds of lymphocytes, T cell and NK cell, in one.

The compound (I) and its pharmaceutically acceptable salt according to this invention have the property to activate or augment NKT cells and, therefore, are particularly useful as autoimmunosuppressant and/or potentiator on normal immune responses through the augmentation of NKT cells. As such, these are effective in the therapy and/or prophylaxis of inflammatory conditions, various types of pain, collagen disease, autoimmune diseases and various immunity diseases, and the like in man and animals, especially in the treatment and/or prevention of inflammation and pain in joint and muscle [e.g. rheumatoid arthritis, rheumatoid spondylitis, osteoarthritis, gouty arthritis, etc.], inflammatory skin condition [e.g. sunburn, eczema, etc.], inflammatory eye condition [e.g. conjunctivitis, etc.], lung disorder in which inflammation is involved [e.g. asthma, bronchitis, pigeon fancier's disease, farmer's lung, etc.], condition of the gastrointestinal tract associated with inflammation [e.g. aphthous ulcer, Crohn's disease, atrophic gastritis, gastritis varialoforme, ulcerative colitis, coeliac disease, regional ileitis, irritable bowel syndrome, etc.], gingivitis, (inflammation, pain and tumescence after operation or injury), pyrexia, pain and other conditions associated with inflammation, rejection by transplantation, systemic lupus erythematosus, scleroderma, polymyositis, polychondritis, periarteritis nodosa, ankylosing spondytis, inflammatory chronic renal condition [e.g. glomerulonephritis, lupus nephritis, membranous nephritis, etc.]. rheumatic fever, Sjögren's syndorome, Behcet disease, thyroiditis, type I diabetes, dermatomyositis, chronic active hepatitis, myasthenia gravis, idiopathic sprue, Grave's disease, multiple sclerosis, primary billiary cirrhoris, Reiter's syndrome, autoimmune hematological disorders [e.g. hemolytic anemia, pure red cell anemia, idiopathic thrombocytopenia, aplastic anemia, etc.], uveitis, contact dermatitis, psoriasis, Kawasaki disease, type I allergy-associated diseases (e.g. allergic asthma, atopic dermatitis, urticaria, allergic conjunctivitis, pollinosis, etc.), shocks (e.g. septic shock, anaphylactic shock, adult respiratory distress syndrome, etc.), sarcoidosis, Wegner's granulomatosis, Hodgkin's disease, cancer [e.g. lung carcinoma, stomach carcinoma, colon cancer, renal carcinoma, hepatoma, etc.], viral diseases [e.g. hepatitis, chronic fatigue syndrome, etc.], and the like.

The compound (I) or pharmaceutically acceptable salt thereof as the active ingredient of this invention can be administered directly as it is, but is generally administered as various pharmaceutical compositions suited for medicament.

The dosage forms of such pharmaceutical compositions include oral dosage forms such as solutions, emulsions, suspensions, capsules, granules, powders, tablets, syrups, etc., external or topical dosage forms such as ointments, eye-drops, nose-drops, etc., parenteral dosage forms, and suppositories.

Such pharmaceutical compositions can be formulated in the routine manner using suitable excipients, such as sucrose, starch, mannit, sorbit, lactose, glucose, cellulose, talc, calcium phosphate, calcium carbonate, etc.; binders, such as cellulose, methylcellulose, hydroxypropylcellulose, polypropylpyrrolidone, gelatin, gum arabic, polyethylene glycol, sucrose, starch, etc.; disintegrators such as starch, carboxymethylcellulose, hydroxypropylstarch, sodium hydrogencarbonate, calcium phosphate, carboxymethylcellulose calcium, calcium citrate, etc.; lubricants, such as magnesium stearate, talc, sodium lauryl sulfate, etc.; corrigents, such as citric acid, menthol, glycine, sorbitol, orange powder, etc.; preservatives, such as sodium benzoate, sodium hydrosulfite, methyl p-hydroxybenzoate, propyl p-hydroxybenzoate, etc.; stabilizers, such as citric acid, sodium citrate, sodium edetate, acetic acid, etc.; suspending agents such as methylcellulose, polyvinylpyrrolidone, aluminum stearate, etc.; dispersants, such as hydroxypropylmethylcellulose etc.; solvents such as water etc.; solubilizers, such as hydrochloric acid etc.; emulsifiers, such as sodium monostearte etc.; a flavorant, such as lemon essence premier etc.; and antiseptics, such as benzalkonium chloride, etc.; and ointment bases such as cacao butter, polyethylene glycol, microcrystalline wax, bleached beeswax, liquid paraffin, white petrolatum, and so on.

The dosage of such a pharmaceutical composition according to this invention is dependent on age, body weight and conditions of the patient or the administering method. Usually, however, the composition is administered orally or otherwise in a unit dose of 0.001∼300 mg/kg, preferably 0.32∼32 mg/kg, as the compound (I) or its pharmaceutically acceptable salt.

The following test examples demonstrate the outstanding NKT cell augmenting effect of the compound and pharmaceutically acceptable salt of this invention and the marked therapeutic efficacy thereof in lupus nephritis which is one of autoimmune diseases.

### Test Example 1: Augmentation of NKT cells

### 1. Method

To W/BF1 mice which develop a spontaneous lupus nephritis, Test compound (10 mg/kg) was orally administered every day during the period of 6∼16 weeks of age. At week 16, the splenocyte was prepared, then, the percentage of NKT cells in the total splenocyte population was determined by flow cytometry after double-staining with anti-TCR antibody and anti-NK1.1 antibody.

### 2. Test compound

### 6-Chloro-1,2-dihydro-4-hydroxy-3-[N-methyl-N-(4-methoxyphenyl)(thiocarbamoyl)]-1-methyl-2-oxoquinoline

### 3. Result

| | Percentage of NKT cells in total spleen cell population |
|---|---|
| Control | 0.48±0.08 |
| Test compound | 1.19*±0.05 |

| | |
|---|---|
| *: Significant difference from control at P<0.01. | |

### Test Example 2: Therapeutic efficacy in lupus nephritis

### 1. Method

To male W/BF1 mice which develop a spontaneous lupus nephritis, Test compound (10 mg/kg) was orally administered every day during the period of 6∼16 weeks of age. The kidneys were then excised and fixed in formalin and pathological specimens were prepared and examined histopathologically for nephritis.

### 2. Test compound

### 6-Chloro-1,2-dihydro-4-hydroxy-3-[N-methyl-N-(4-methoxyphenyl)(thiocarbamoyl)]-1-methyl-2-oxoquinoline

### 3. Result

| | Histopathological evaluation of nephritis |
|---|---|
| Control | 2.80±0.49 |
| Test compound | 0.50*±0.22 |

| | |
|---|---|
| *: Significant difference from control at P<0.01. | |

### Test Example 3: Toxicity test

Test compound (10 mg/kg) was administered to rats (5 animals of either sex per group) once daily, 7 days a week, for 2 consecutive weeks but no death was encountered during the period.

The following Preparations and Examples are given for the purpose of illustrating the present invention in more detail.

### Preparation 1-(1)

To a solution of N-methylaniline (27 ml) and pyridine (40.3 ml) in dichloromethane (270 ml), acetyl chloride (23.0 ml) was added dropwise at 0°C, and the mixture was stirred at the same temperature for 15 minutes. This reaction mixture was diluted with water (300 ml) and, after phase separation, the organic layer was washed with 1 N-hydrochloric acid (300 ml) twice. It was further successively washed with aqueous sodium hydrogencarbonate solution and brine and dried over magnesium sulfate and the solvent was then evaporated. The residue was recrystallized from diisopropyl ether to provide N-phenyl-N-methylacetamide (33.5 g; yield 90.2%) as colorless crystals.
m.p. 97∼98°C

### Preparation 1-(2)

A mixture of N-phenyl-N-methylacetamide (13.9 g) and phosphorus pentasulfide (4.2 g) in toluene was stirred at 110°C for 3 hours. This reaction mixture was poured in a mixture of sodium carbonate, water and dichloromethane. The whole mixture was filtered through Celite and the filtrate was separated. The organic layer was dried over magnesium sulfate and, then, evaporated. The residue was purified by column chromatography on silica gel (250 g; n-hexane:ethyl acetate = 10:1) to provide N-phenyl-N-methyl(thioacetamide) (11.3 g; yield 73.2%).
m.p. 53∼54°C

### Preparation 2

A mixture of N-(4-methoxyphenyl)-N-methylacetamide (96.9 g) and phosphorus pentasulfide (31.3 g) in toluene was stirred at 110°C for 3 hours. This reaction mixture was poured in a mixture of sodium carbonate, water and dichloromethane. The whole mixture was filtered through Celite and the filtrate was separated. The organic layer was dried over magnesium sulfate and, then, evaporated. The residue was purified by column chromatography on silica gel (620 g; n-hexane:ethyl acetate = 10:1) to provide N-(4-methoxyphenyl)-N-methyl(thioacetamide) (103 g).
NMR (CDCl₃, δ): 2.40 (3H, s), 3.72 (3H, s), 3.84 (3H, s), 6.9-7.2 (4H, m)
Mass (m/z): 196 (m+1)⁺

### Preparation 3

Under nitrogen at 0°C, butyllithium (1.53 M in hexane) (11.4 ml) was added dropwise to a solution of diisopropylamine (2.8 ml) in tetrahydrofuran (50 ml). This mixture was stirred for 10 minutes and cooled to -20°C. Then, N-phenyl-N-methyl(thioacetamide) (2.0 g) was added and the whole mixture was stirred at -20°C for 80 minutes. To this was added dry ice (3 g), and the mixture was stirred at -20°C for 40 minutes. To this reaction mixture was added isopropyl ether (50 ml) and water (50 ml). The aqueous layer was mixed with dichloromethane (50 ml) and the mixture was acidified with 4 N-hydrochloric acid (10 ml) at 5°C. The organic layer was separated, dried and evaporated. The residue was stirred in toluene (10 ml)-hexane (8 ml) at -15°C for 1 hour and the resulting precipitate was collected and washed with a cold mixture of hexane and toluene (1:1) to provide 3-(N-phenyl-N-methylamino)-3-thioxopropionic acid (1.68 g; yield 66.4%) as white crystals.
IR (Nujol): 3450, 1715, 1590 cm⁻¹
NMR (CDCl₃, δ): 3.70 (2H, s), 3.77 (3H, s), 7.1-7.6 (5H, m)

### Preparation 4

Under nitrogen at 0°C, butyllithium (1.53 M in hexane) (506 ml) was added dropwise to a solution of diisopropylamine (124 ml) in tetrahydrofuran (1.01). This mixture was stirred for 10 minutes and cooled to -20°C. Then, N-(4-methoxyphenyl)-N-methyl(thio-acetamide) (105 g) was added dropwise and the whole mixture was stirred at -20°C for 80 minutes. Then, dry ice (132 g) was added dropwise and the mixture was stirred at -20°C for 40 minutes. This reaction mixture was diluted with isopropyl ether (400 ml) and water (1 l). The aqueous layer was mixed with dichloromethane (1.3 l) and the mixture was acidified with concentrated hydrochloric acid (157 ml) at 5°C. The organic layer was separated, dried and evaporated. The residue was stirred in toluene (307 ml)-hexane (244 ml) at -15°C for 1 hour and the resulting precipitate was washed with a cold mixture of hexane and toluene (1:1) to provide 3-[N-(4-methoxyphenyl)-N-methylamino]-3-thioxopropionic acid (81.2 g) as light-brown crystals.
NMR (CDCl₃, δ): 3.70 (2H, s), 3.74 (3H, s), 3.85 (3H, s), 6.9-7.2 (4H, m)

### Preparation 5

Under nitrogen at room temperature, a mixture of 3-(N-phenyl-N-methylamino)-3-thioxopropionic acid (25.3 g), powdery molecular sieves (4Å, activated form, Aldrich; 12.7 g) and pyridine (19.5 ml) in dichloromethane (250 ml) was stirred for 30 minutes, and then cooled to -20°C. Pivaloyl chloride (14.9 ml) was added dropwise and the whole mixture was stirred at -20°C for 30 minutes. Then, ethyl 5-chloro-2-(methylamino)benzoate (21.6 g) was added and the mixture was stirred at -20°C for 1 minute, at 0°C for 1 hour, and then at room temperature overnight. This mixture was poured in a mixture of ethyl acetate (1 l) and brine (600 ml) and the whole mixture was filtered through Celite. The organic layer was separated, washed successively with diluted hydrochloric acid, brine and aqueous sodium hydrogencarbonate solution, dried, and evaporated. The residue was subjected to silica gel (500 g) column chromatography (hexane:ether = 1:2) to provide ethyl 5-chloro-2-[N-[3-(N'-phenyl-N'-methylamino)-3-thioxopropionyl]-N-methylamino]benzoate (35.2 g; yield 86.0%).
NMR (CDCl₃, δ): 1.20 (3H, t, J=7 Hz), 3.12 (3H, s), 3.35 (2H, s), 3.71 (3H, s), 4.20 (2H, q, J=7 Hz), 7.1-7.5 (7H, m), 7.87 (1H, d, J=2.6 Hz)
Mass (m/z): 405 (M+1)⁺

### Preparation 6

Under nitrogen at room temperature, a mixture of 3-[N'-(4-methoxyphenyl)-N'-methylamino]-3-thioxopropionic acid (30.8 g), powdery molecular sieves (4Å, activated form, Aldrich) and pyridine (18.1 ml) in dichloromethane (234 ml) was stirred for 30 minutes, and then cooled to -20°C. Pivaloyl chloride (13.8 ml) was added dropwise and the mixture was stirred at - 20°C for 30 minutes. Then, ethyl 5-chloro-2-(methylamino)benzoate (20 g) was added and the mixture was stirred at -20°C for 1 minute, at 0°C for 1 hour, and then at room temperature overnight. This mixture was poured in a mixture of ethyl acetate (1 l) and brine (600 ml) and the whole mixture was filtered through Celite. The organic layer was separated, washed successively with diluted hydrochloric acid, brine and aqueous sodium hydrogencarbonate solution, dried, and evaporated. The residue (64 g) was subjected to silica gel (480 g) column chromatography (hexane:ether = 1:2) to provide ethyl 5-chloro-2-[N-[3-[N'-(4-methoxyphenyl)-N'-methylamino]-3-thioxopropionyl]-N-methylamino]benzoate (33.7 g).
NMR (CDCl₃, δ): 1.22 (3H, t, J=7 Hz), 3.14 (3H, s), 3.36 (2H, s), 3.68 (3H, s), 3.83 (3H, s), 4.20 (2H, q, J=7 Hz), 6.9-7.6 (6H, m), 7.88 (1H, d, J=2.5 Hz)
Mass (m/z): 435 (M+1)⁺, 222

### Example 1

Sodium methoxide (7.70 g) was added to an ice-cooled suspension of ethyl 5-chloro-2-[N-[3-(N'-phenyl-N'-methylamino)-3-thioxopropionyl]-N-methylamino]benzoate (35.2 g) in ethanol (434 ml) and the resulting mixture was stirred at 5°C for 40 minutes and then poured into ice water (2.2 l). To this was added 3 N-hydrochloric acid (43 ml), and the resulting precipitate was collected and washed with water to provide 6-chloro-1,2-dihydro-4-hydroxy-1-methyl-3-[(N-phenyl-N-methyl(thiocarbamoyl)]-2-oxoquinoline (30.2 g; yield 96.9%).
m.p. 218°C (decompn.)
IR (Nujol): 3150, 1625, 1590, 1570, 1495 cm⁻¹
NMR (DMSO-d₆, δ): 3.42 (3H, s), 3.74 (3H, s), 7.1-7.7 (7H, m), 7.81 (1H, d, J=2 Hz), 11.15 (1H, s)

### Example 2

Sodium methoxide (6.87 g) was added to an ice-cooled suspension of ethyl 5-chloro-2-[N-[3-[N'-(4-methoxyphenyl)-N'-methylamino]-3-thioxopropionyl]-N-methylamino]benzoate (33.7 g) in ethanol (390 ml) and the resulting mixture was stirred at 5°C for 40 minutes and poured into ice water (1.8 l). To this was added 3 N-hydrochloric acid (36 ml), and the resulting precipitate was collected and washed with water to provide 6-chloro-1,2-dihydro-4-hydroxy-1-methyl-3-[N-(4-methoxyphenyl)-N-methyl(thiocarbamoyl)]-2-oxoquinoline (32.4 g).
m.p. 195°C (decompn.)
IR (KBr): 1629, 1606, 1571, 1508 cm⁻¹
NMR (DMSO-d₆, δ): 3.43 (3H, s), 3.63 (3H, s), 3.69 (3H, s), 6.79 (2H, d, J=9 Hz), 7.2-7.6 (4H, m), 7.82 (1H, d, J=2 Hz), 11.12, (1H, s) Mass (m/z): 389 (M+1)⁺

### Example 3

The components of the following formulation were mixed and compressed in the routine manner to provide tablets.

| Tablet prescription | | |
|---|---|---|
| Active component | Test compound | 32 mg |
| Disintegrator | Carboxymethylcellulose calcium | 6 mg |
| Binder | Hydroxypropylcellulose | 2 mg |
| Excipient | Crystalline cellulose | suitable amount |
| Lubricant | Magnesium stearate | 5 mg |
| Total | | 90 mg |

### Example 4

The components of the following formulation were mixed in the routine manner to provide an ointment.

| Ointment prescription | | |
|---|---|---|
| Active component | Test compound | 5 g |
| Base | Bleached beeswax | 5 g |
| Base | Liquid paraffin | 24 g |
| Base | White petrolatum | 40 g |
| Base | Microcrystalline wax | 2 g |
| Emulsifier | Glycerin monostearate | 2 g |
| Solvent | Purified water | suitable amount |
| Total | | 100 g |

## Claims

1. A NKT cell augmentation-mediated autoimmunosuppressant and/or potentiator on normal immune responses characterized by comprising a compound of the following general formula or a pharmaceutically acceptable salt thereof as an active ingredient. 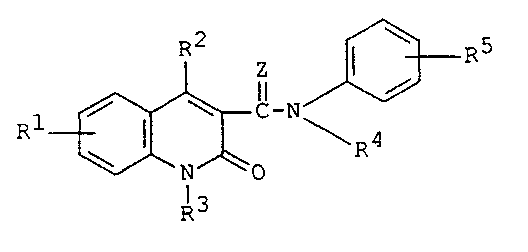
[wherein R¹ represents hydrogen or halogen,
R² represents hydroxy,
R³ represents lower alkyl,
R⁴ represents lower alkyl,
R⁵ represents hydrogen or lower alkoxy, and
Z represents O or S]

2. A NKT cell augmentation-mediated autoimmunosuppressant characterized by comprising the compound according to claim 1 or pharmaceutically acceptable salt thereof as an active ingredient.

3. A NKT cell augmentation-mediated autoimmunosuppressant according to Claim 2 comprising the compound wherein R¹ is halogen,
R² is hydroxy,
R³ is lower alkyl,
R⁴ is lower alkyl,
R⁵ is hydrogen or lower alkoxy, and
Z is S or a pharmaceutically acceptable salt thereof.

4. A NKT cell augmentation-mediated autoimmunosuppressant according to Claim 2 comprising the compound wherein R¹ is hydrogen,
R² is hydroxy,
R³ is lower alkyl,
R⁴ is lower alkyl,
R⁵ is hydrogen, and
Z is O or a pharmaceutically acceptable salt thereof.

5. A NKT cell augmentation-mediated autoimmunosuppressant according to Claim 3 in which the compound is selected from the group consisting of (1) 6-chloro-1,2-dihydro-4-hydroxy-1-methyl-2-oxo-3-[N-phenyl-N-methyl(thiocarbamoyl)]quinoline or a pharmaceutically acceptable salt thereof and (2) 6-chloro-1,2-dihydro-4-hydroxy-1-methyl-2-oxo-3-[N-(4-methoxy)phenyl-N-methyl(thiocarbamoyl)]quinoline or a pharmaceutically acceptable salt thereof.

6. A NKT cell augmentation-mediated autoimmunosuppressant according to Claim 4 in which the compound is 1,2-dihydro-4-hydroxy-1-methyl-2-oxo-3-(N-phenyl-N-methylcarbamoyl)quinoline or a pharmaceutically acceptable salt thereof.

7. A NKT cell augmentation-mediated potentiator on normal immune responses characterized by comprising the compound of claim 1 or pharmaceutically acceptable salt thereof.

8. A NKT cell augmentation-mediated potentiator on normal immune responses according to Claim 7 comprising the compound wherein R¹ is halogen,
R² is hydroxy,
R³ is lower alkyl,
R⁴ is lower alkyl,
R³ is hydrogen or lower alkoxy, and
Z is S or a pharmaceutically acceptable salt thereof.

9. A NKT cell augmentation-mediated potentiator on normal immune responses according to Claim 7 comprising the compound wherein R¹ is hydrogen,
R² is hydroxy,
R³ is lower alkyl,
R⁴ is lower alkyl,
R⁵ is hydrogen and
Z is O or a pharmaceutically acceptable salt thereof.

10. A NKT cell augmentation-mediated potentiator on normal immune responses according to Claim 8 in which the compound is selected from the group consisting of (1) 6-chloro-1,2-dihydro-4-hydroxy-1-methyl-2-oxo-3-[N-phenyl-N-methyl(thiocarbamoyl)]quinoline or a pharmaceutically acceptable salt thereof and (2) 6-chloro-1,2-dihydro-4-hydroxy-1-methyl-2-oxo-3-[N-(4-methoxy)phenyl-N-methyl(thiocarbamoyl)]quinoline or a pharmaceutically acceptable salt thereof.

11. A NKT cell augmentation-mediated potentiator on normal immune responses according to Claim 9 in which the compound is 1,2-dihydro-4-hydroxy-1-methyl-2-oxo-3-(N-phenyl-N-methylcarbamoyl)quinoline or a pharmaceutically acceptable salt thereof.

12. A process for producing a compound of the following general formula: 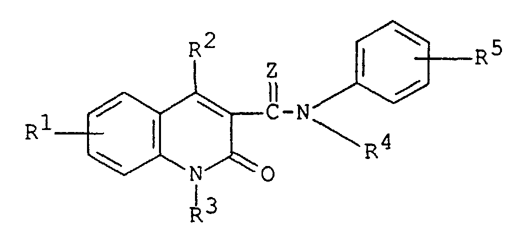
[wherein R¹ represents hydrogen or halogen,
R² represents hydroxy,
R³ represents lower alkyl,
R⁴ represents lower alkyl,
R⁵ represents hydrogen or lower alkoxy, and
Z represents O or S] or a salt thereof, which comprises subjecting the following general formula: 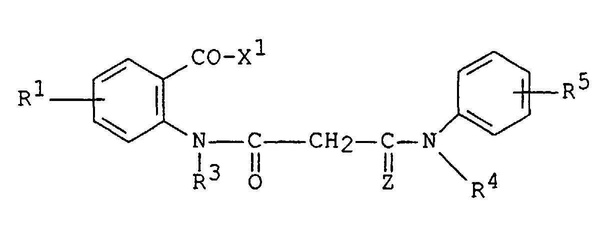
[wherein R¹ , R³ , R⁴ , R⁵ and Z are each as defined above,
and, X¹ represents a leaving group] or a salt thereof to cyclization reaction.

13. A compound of the following general formula: 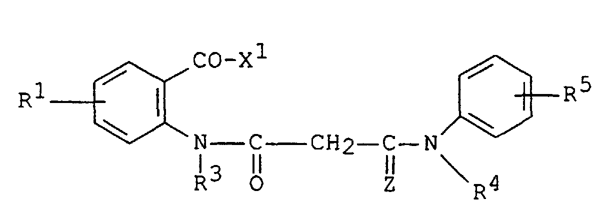
[wherein R¹ represents hydrogen or halogen,
R³ represents lower alkyl,
R⁴ represents lower alkyl,
R⁵ represents hydrogen or lower alkoxy,
X¹ represents a leaving group, and
Z represents O or S] or a salt thereof.

14. A compound of Claim 13, wherein
R¹ represents halogen,
R⁵ represents hydrogen or lower alkoxy, and
Z represents S.

15. A compound of Claim 14, which is selected from the group consisting of
1. ethyl 5-chloro-2-[N-[3-(N-phenyl-N-methyl-amino)-3-thioxopropionyl]-N-methylamino]benzoate and
2. ethyl 5-chloro-2-[N-[3-[N-(4-methoxyphenyl)-N-methylamino]-3-thioxopropionyl]-N-methyl-amino]benzoate.

16. A process for producing a compound of the following general formula: 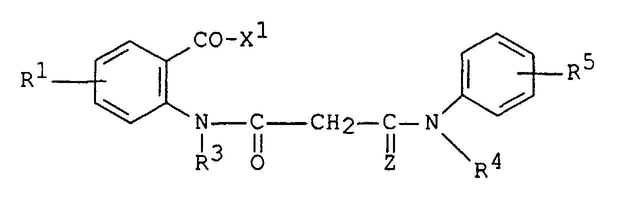
[wherein R¹ represents hydrogen or halogen,
R³ represents lower alkyl,
R⁴ represents lower alkyl,
R⁵ represents hydrogen or lower alkoxy,
X¹ represents a leaving group, and
Z represents O or S] or a salt thereof which comprises reacting a compound of the general formula 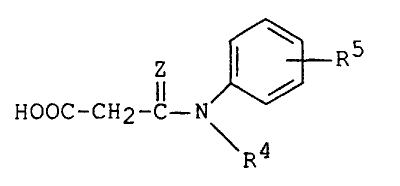
[wherein R⁴ , R⁵ and Z are each as defined above] or a reactive derivative at its carboxyl group thereof, or a salt thereof, with a compound of the general formula 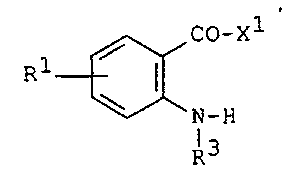
[wherein R¹ , R³ and X¹ are each as defined above] or a reactive derivative of its imino group thereof, or a salt thereof.
